(19) Europäisches Patentamt / European Patent Office / Office européen des brevets

(11) **EP 4 772 191 A1**

(12) **EUROPEAN PATENT APPLICATION**
published in accordance with Art. 153(4) EPC

(43) Date of publication:
**08.07.2026 Bulletin 2026/28**

(21) Application number: **23950580.3**

(22) Date of filing: **29.12.2023**

(51) International Patent Classification (IPC):
**A61K 39/395** *(2006.01)*    **A61K 31/436** *(2006.01)*
**A61K 9/107** *(2006.01)*    **A61K 9/127** *(2025.01)*
**A61P 35/00** *(2006.01)*

(86) International application number:
**PCT/CN2023/143213**

(87) International publication number:
**WO 2025/044015 (06.03.2025 Gazette 2025/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **01.09.2023 CN 202311128066**

(71) Applicant: **GUANGZHOU PENGXING MEDICAL TECHNOLOGY CO., LTD.**
**Guangzhou, Guangdong 510145 (CN)**

(72) Inventor: **YAN, Pengke**
**Guangzhou, Guangdong 510145 (CN)**

(74) Representative: **Barker Brettell LLP**
**100 Hagley Road**
**Edgbaston**
**Birmingham, West Midlands B16 8QQ (GB)**

(54) **USE OF RAPAMYCIN COMBINED WITH PD-1 MONOCLONAL ANTIBODY IN PREPARATION OF DRUGS FOR TREATING COLORECTAL CANCER**

(57)    Provided are a use of rapamycin in combination with PD-1 monoclonal antibody in preparation of drugs for treating colorectal cancer, and a corresponding pharmaceutical composition. The tumor immune microenvironment is remodeled by means of a rapamycin formulation targeting tumor tissues, the infiltration of T lymphocytes in tumor tissues is increased, and M2 related TAMs are inhibited, thereby enhancing the anti-tumor, especially anti-microsatellite stable colorectal cancer effect of the PD-1 monoclonal antibody.

FIG.4

Processed by Luminess, 75001 PARIS (FR)

EP 4 772 191 A1

**Description**

**Cross reference to related applications**

This application claims the priority of the Chinese patent application filed with the China National Intellectual Property Administration on September 1, 2023, with application number 202311128066.4 and title "Use of Rapamycin Combined with PD-1 Monoclonal Antibody in preparation of drugs for treating colorectal cancer", the entire content of which is incorporated herein by reference.

**TECHNICAL FIELD**

The present application belongs to the field of tumor therapy. Specifically, the present application provides the use of rapamycin combined with a PD-1 monoclonal antibody in preparation of drugs for treating colorectal cancer, as well as corresponding pharmaceutical compositions.

**BACKGROUND**

Colorectal cancer (CRC) has become one of the three most common malignant tumors in China. With continuous advances in medical diagnosis and treatment, the therapy of malignant tumors has entered the era of immunotherapy. Currently, based on populations, PD-1 inhibitors used for treating colorectal cancer are divided into two, microsatellite instability (MSI) or mismatch repair gene status (MMR) status are the best predictive markers for efficacy, and colorectal cancer patients are classified into two groups:

"favorable population": MSI-H/dMMR type of colorectal cancer (abbreviated as MSI-H type of colorectal cancer);
"non-responsive population": MSS/pMMR type of colorectal cancer (abbreviated as MSS type of colorectal cancer).

For patients with MSI-H advanced CRC, PD-1 monoclonal antibodies (pembrolizumab, KeyTruda, nivolumab, Opdivo) are strongly recommended as first-line regimens in both NCCN guidelines and CSCO guidelines. However, studies on immunotherapy for patients with MSS type of advanced CRC have not achieved satisfactory results. This type of CRC patients accounts for more than 95% of all CRC patients. A sharp contradiction exists between the urgent and strong medical demand and the relatively limited therapeutic approaches, and this therapeutic dilemma urgently needs to be solved.
At present, the core strategy to convert MSS type of colorectal cancer which is a "cold tumor" resistant to immunotherapy into a "hot tumor" responsive to immunotherapy is combination therapy, and transforming patients initially unresponsive for PD-1 inhibitor therapy into beneficiaries of the PD-1 inhibitor therapy can also improve therapeutic efficacy. Current mainstream combination partners include:

(1) Combination with another immunotherapeutic agent: PD-1 inhibitor combined with CTLA-4 antibody, already approved for malignant melanoma,
(2) Combination with chemotherapy: PD-1 inhibitor combined with chemotherapy, approved for first-line treatment of advanced non-squamous non-small cell lung cancer; similar regimens having shown promising preliminary data in gastric cancer, intestinal cancer, triple-negative breast cancer, etc,
(3) Combination with radiotherapy: PD-1 inhibitor combined with radiotherapy in tumors such as lung cancer, malignant melanoma,
(4) Combination with targeted agents: PD-1 inhibitor combined with anti-angiogenic targeted drugs or EGFR inhibitors, with caution due to potential severe side effects.

The mammalian target of rapamycin (mTOR) signaling pathway is a major regulator of cell growth and metabolism, existing in two functionally distinct complexes: mTORC1 and mTORC2. The mTORC1 (mTOR complex 1) signaling pathway is critical for cell growth and proliferation, and is involved in regulating autophagy, ribosome biogenesis, mRNA translation and metabolism. Dysregulated activation of the mTORC1 signaling pathway is highly prevalent in malignant tumors. Therefore, dugs targeting mTORC1 represent a promising anticancer strategy.
Rapamycin as a targeted drug and an mTOR inhibitor has good application prospects in tumor therapy. Our previous studies found that a rapamycin formulation combined with 5-fluorouracil exert a positive synergistic effect in the treatment of colorectal cancer. Accordingly, we consider combining rapamycin liposomes (Rapa LPs) with immunosuppressants to see if it can improve the tumor microenvironment, thereby exerting a positive synergistic or promoting effect in the treatment of colorectal cancer.

**SUMMARY**

To further develop such combination regimens, in one aspect, the present application provides the use of rapamycin combined with a PD-1 monoclonal antibody in preparation of drugs for treating colorectal cancer.

Optionally, rapamycin and the PD-1 monoclonal antibody are formulated in a single pharmaceutical composition, or rapamycin and the PD-1 monoclonal antibody are used in combination.

Optionally, rapamycin in the drugs for treating colorectal cancer exists in a form selected from the group consisting of liposomes, micelles, and a self-microemulsifying solution.

Optionally, the preparation method of the self-microemulsifying solution comprises:

(1) mixing two or more components selected from anhydrous ethanol, propylene glycol, polyethylene glycol 300, and polyethylene glycol 400, adding rapamycin, and optionally one or more antioxidants selected from 2,6-di-tert-butyl-4-methylphenol, bis(3,5-di-tert-butyl-4-hydroxyphenyl)sulfide, tetrakis[$\beta$-(3,5-di-tert-butyl-4-hydroxyphenyl)propionic acid] pentaerythritol ester, BHT, $\alpha$-tocopherol, thioglycerol, and BHA, to prepare a stock solution;

(2) preparing a diluent by using one or more surfactants, anhydrous ethanol, water, and optionally one or more metal chelating agents, wherein the surfactants are selected from polyethylene glycol stearates such as PEG-10 hydroxystearate, PEG-12 hydroxystearate, PEG-18 stearate, PEG-10 stearate, and PEG-15 stearate, and the metal chelating agents are selected from ethylenediaminetetraacetic acid, nitrilotriacetic acid, diethylenetriaminepentaacetic acid and salts thereof, citric acid, tartaric acid, gluconic acid, hydroxyethylethylenediaminetriacetic acid, polyacrylic acid, polymethacrylic acid, hydrolyzed polymaleic anhydride, fumaric acid (trans-butenedioic acid) -allyl sulfonic acid copolymer, dihydroxyethylglycine, and sodium calcium edetate;

(3) mixing the drug stock solution and the diluent to obtain the self-microemulsifying solution.

Optionally, the preparation method of the self-microemulsifying solution comprises:

(1) mixing anhydrous ethanol and polyethylene glycol 300 solution uniformly to obtain a mixture solution, adding rapamycin to the mixture solution, and then adding an antioxidant and anhydrous citric acid to obtain a drug stock solution;

(2) preparing a diluent: measuring polyethylene glycol-10 hydroxystearate, anhydrous ethanol, and water for injection, mixing uniformly, then adding a metal chelating agent, and mixing uniformly to obtain a diluent;

(3) diluting the drug stock solution to a desired concentration with the diluent to obtain a rapamycin self-microemulsifying injection.

Optionally, the preparation method of the self-microemulsifying solution comprises:

(1) mixing 0.5 ml of anhydrous ethanol and 0.5 ml of polyethylene glycol 300 uniformly to obtain a mixture solution, adding rapamycin to the mixture solution, and then adding 0.5 $\mu$g of antioxidant and 2.5 $\mu$g of anhydrous citric acid to obtain a 20 mg/ml drug stock solution;

(2) preparing a diluent: measuring 0.8 ml of polyethylene glycol-10 hydroxystearate, 0.2 ml of anhydrous ethanol, and 1.0 ml of water for injection, mixing uniformly, then adding 0.004 g of a metal chelating agent, and mixing uniformly to obtain a diluent;

(3) filtering and sterilizing the drug stock solution and the diluent separately, followed by hermetically storing them at 4 °C, and diluting the drug stock solution to a desired concentration with the diluent prior to use to obtain a rapamycin self-microemulsifying injection.

Optionally, the PD-1 monoclonal antibody is selected from pembrolizumab, nivolumab, sintilimab, toripalimab, tislelizumab, camrelizumab and anti-mouse PD-1 CD279 monoclonal antibody from BioXcell, inVivoMab.

Optionally, the colorectal cancer is microsatellite stable colorectal cancer.

Optionally, rapamycin is combined with the PD-1 monoclonal antibody in the drug.

Optionally, the weight ratio of rapamycin to the PD-1 monoclonal antibody in the drugs is 1:1 to 4:1.

Optionally, the weight ratio of rapamycin to the PD-1 monoclonal antibody in the drugs is 1:1 to 2:1.

Optionally, the weight ratio of rapamycin to the PD-1 monoclonal antibody in the drugs is 1:1 to 1:20.

In another aspect, the present application provides a drug for treating colorectal cancer comprising rapamycin and a PD-1 monoclonal antibody; rapamycin and the PD-1 monoclonal antibody are formulated in a single pharmaceutical composition, or rapamycin and the PD-1 monoclonal antibody are provided separately.

When used in combination, the dosage of rapamycin is 0.5-4 mg/kg, and the dosage of the PD-1 monoclonal antibody is 0.5-80 mg/kg.

The present application also provides a method of treating colorectal cancer, comprising administering to a subject in need thereof rapamycin in combination with a PD-1 monoclonal antibody.

Optionally, rapamycin and the PD-1 monoclonal antibody are formulated in a single pharmaceutical composition, or rapamycin and the PD-1 monoclonal antibody are used in combination.

Optionally, the PD-1 monoclonal antibody is selected from pembrolizumab, nivolumab, sintilimab, toripalimab, tislelizumab, camrelizumab and anti-mouse PD-1 CD279 monoclonal antibody from BioXcell, inVivoMab.

Optionally, in the drug for treating colorectal cancer, rapamycin exists in a form selected from the group consisting of liposomes, micelles, and a self-microemulsifying solution.

Optionally, the preparation method of the self-microemulsifying solution comprises:

(1) mixing two or more components selected from anhydrous ethanol, propylene glycol, polyethylene glycol 300, and polyethylene glycol 400, adding rapamycin, and optionally one or more antioxidants selected from 2,6-di-tert-butyl-4-methylphenol, bis(3,5-di-tert-butyl-4-hydroxyphenyl)sulfide, tetrakis[β-(3,5-di-tert-butyl-4-hydroxyphenyl)propionic acid] pentaerythritol ester, BHT, α-tocopherol, thioglycerol, and BHA, to prepare a stock solution;

(2) preparing a diluent by using one or more surfactants, anhydrous ethanol, water, and optionally one or more metal chelating agents, wherein the surfactants are selected from polyethylene glycol stearates such as PEG-10 hydroxystearate, PEG-12 hydroxystearate, PEG-18 stearate, PEG-10 stearate, and PEG-15 stearate, and the metal chelating agents are selected from ethylenediaminetetraacetic acid, nitrilotriacetic acid, diethylenetriaminepentaacetic acid and salts thereof, citric acid, tartaric acid, gluconic acid, hydroxyethylethylenediaminetriacetic acid, polyacrylic acid, polymethacrylic acid, hydrolyzed polymaleic anhydride, fumaric acid (trans-butenedioic acid) -allyl sulfonic acid copolymer, dihydroxyethylglycine, and sodium calcium edetate;

(3) mixing the drug stock solution and the diluent to obtain the self-microemulsifying solution. Optionally, the preparation method of the self-microemulsifying solution comprises:

(1) mixing anhydrous ethanol and polyethylene glycol 300 solution uniformly to obtain a mixture solution, adding rapamycin to the mixture solution, and then adding an antioxidant and anhydrous citric acid to obtain a drug stock solution;

(2) preparing a diluent: measuring polyethylene glycol-10 hydroxystearate, anhydrous ethanol, and water for injection, mixing uniformly, then adding a metal chelating agent, and mixing uniformly to obtain a diluent;

(3) diluting the drug stock solution to a desired concentration with the diluent to obtain a rapamycin self-microemulsifying injection.

Optionally, the preparation method of the self-microemulsifying solution comprises:

(1) mixing 0.5 ml of anhydrous ethanol and 0.5 ml of polyethylene glycol 300 uniformly to obtain a mixture solution, adding rapamycin to the mixture solution, and then adding 0.5 μg of antioxidant and 2.5 μg of anhydrous citric acid to obtain a 20 mg/ml drug stock solution;

(2) preparing a diluent: measuring 0.8 ml of polyethylene glycol-10 hydroxystearate, 0.2 ml of anhydrous ethanol, and 1.0 ml of water for injection, mixing uniformly, then adding 0.004 g of a metal chelating agent, and mixing uniformly to obtain a diluent;

(3) filtering and sterilizing the drug stock solution and the diluent separately, followed by hermetically storing them at 4 °C, and diluting the drug stock solution to a desired concentration with the diluent prior to use to obtain a rapamycin self-microemulsifying injection.

Optionally, the colorectal cancer is microsatellite stable colorectal cancer.

Optionally, the weight ratio of rapamycin to the PD-1 monoclonal antibody in the drug is 1:1 to 4:1.

Optionally, the weight ratio of rapamycin to the PD-1 monoclonal antibody in the drug is 1:1 to 2:1.

Optionally, the weight ratio of rapamycin to the PD-1 monoclonal antibody in the drug is 1:1 to 1:20.

Any PD-1 monoclonal antibody currently commercially available or under development may be used in the technical solutions of the present application after appropriate experimentation and selection.

The rapamycin formulation in the present application may be prepared by various methods known in the art, which are not limited to the methods in the examples. For reference, the preparation methods disclosed in Chinese patent applications Nos. 201910918643.7, 202011065631.3 and 202210106827.5 may be used.

Each component in the drug of the present application may be in any clinically acceptable dosage form, including various oral and parenteral dosage forms. For oral administration, the dosage forms may be tablets, capsules, soft capsules, oral solutions, syrups, granules, dropping pills, orally disintegrating tablets, sustained-release tablets, sustained-release capsules, controlled-release tablets, controlled-release capsules, etc. For parenteral administration, the dosage forms may be liquid injections, lyophilized powder injections, sterile powder injections, infusions, etc.

Those skilled in the art may use various pharmaceutically acceptable carriers or excipients, such as solvents, cosolvents, pH regulators, osmotic pressure regulators, stabilizers, antioxidants, fillers, adhesives, lubricants, disintegrants, coating agents, sustained-release agents, controlled-release agents, adsorption carriers, etc., to formulate the above dosage forms.

The present application provides a novel method for treating microsatellite stable colorectal cancer, which comprises the use of rapamycin in combination with immunotherapy for colorectal cancer, specifically a PD-1 monoclonal antibody combined with a rapamycin formulation, wherein the tumor immune microenvironment is remodeled by the rapamycin formulation targeting tumor tissue, T-lymphocyte infiltration in tumor tissue is increased, and M2-associated TAMs is inhibited, thereby the anti-tumor effect of the PD-1 monoclonal antibody, especially anti-microsatellite stable colorectal cancer is enhanced.

**Brief Description of the Drawings**

Figure 1 shows the results of the in vitro cell MTT assay.

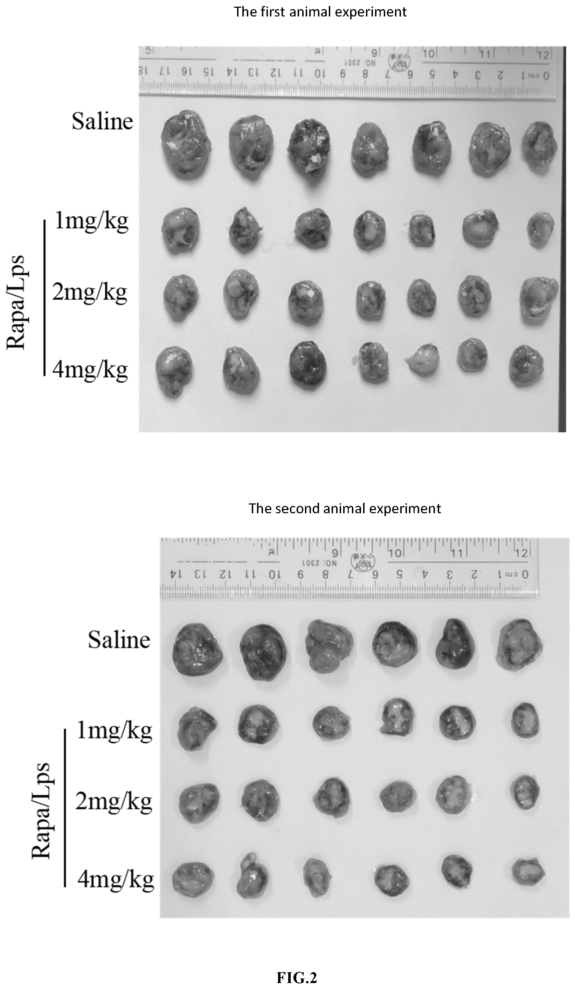

Figure 2 shows the pharmacodynamic results of different concentrations of RL in CT-26 tumor-bearing mice.

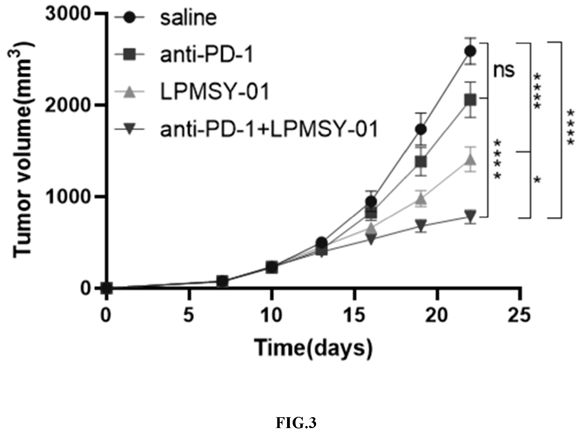

Figure 3 shows the growth curve of transplanted tumors.

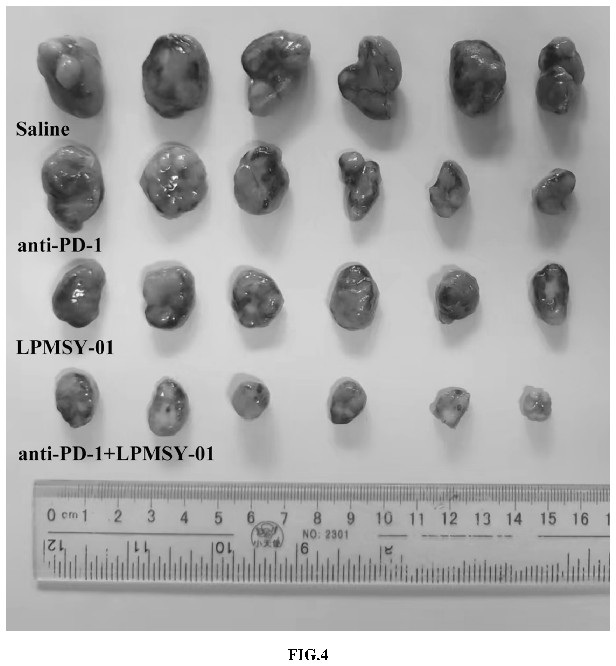

Figure 4 shows the comparison of tumor size in different tumor treatment regimens.

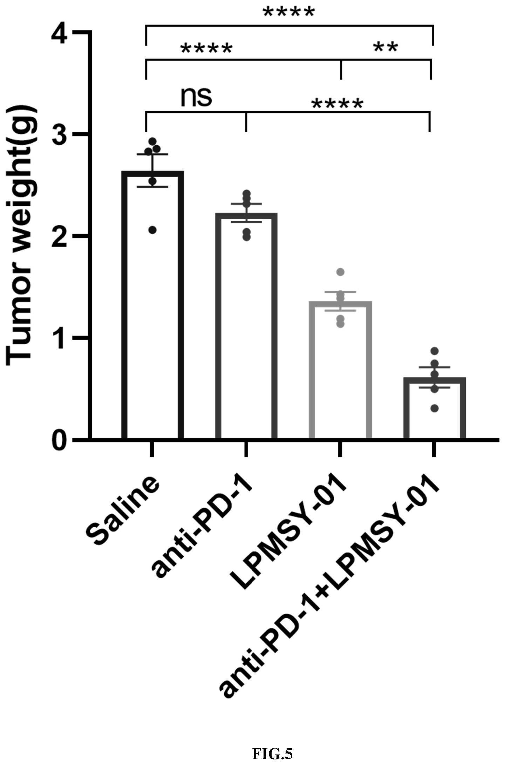

Figure 5 shows the statistical analysis of tumor weight in different tumor treatment regimens.

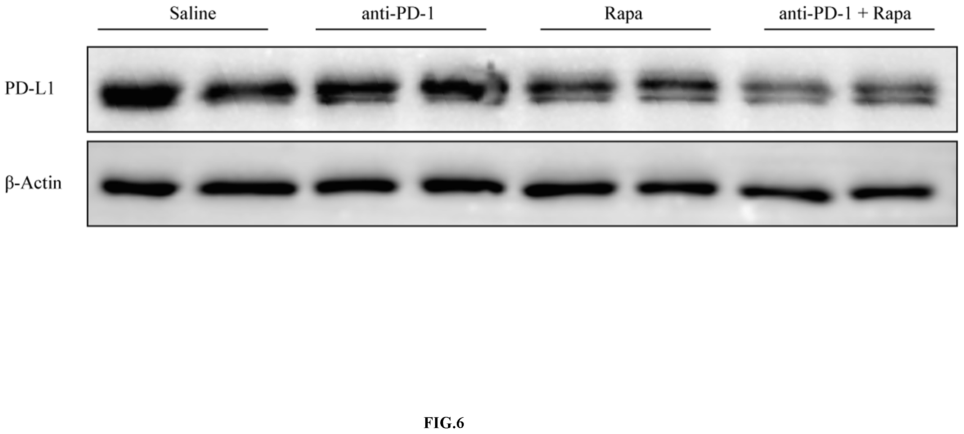

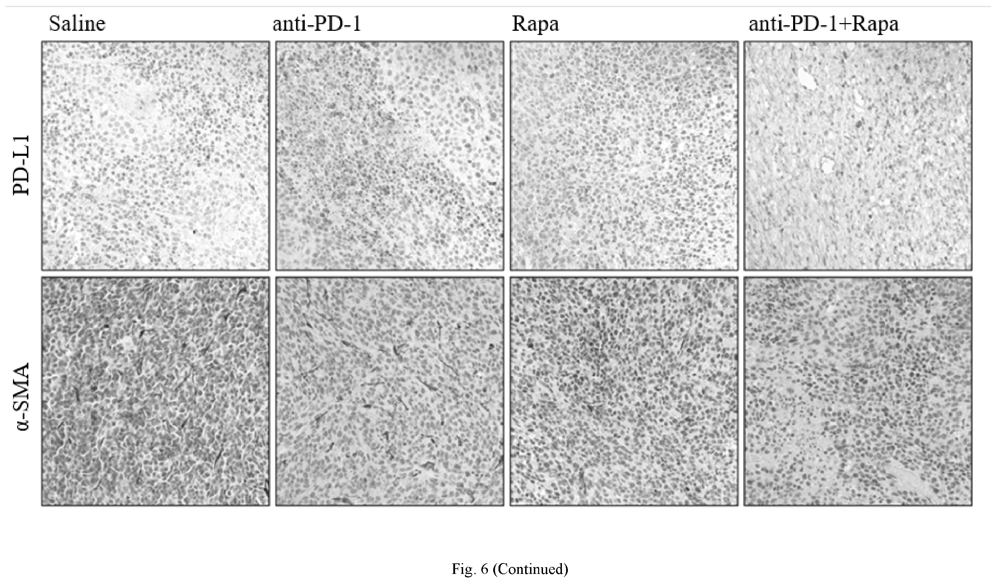

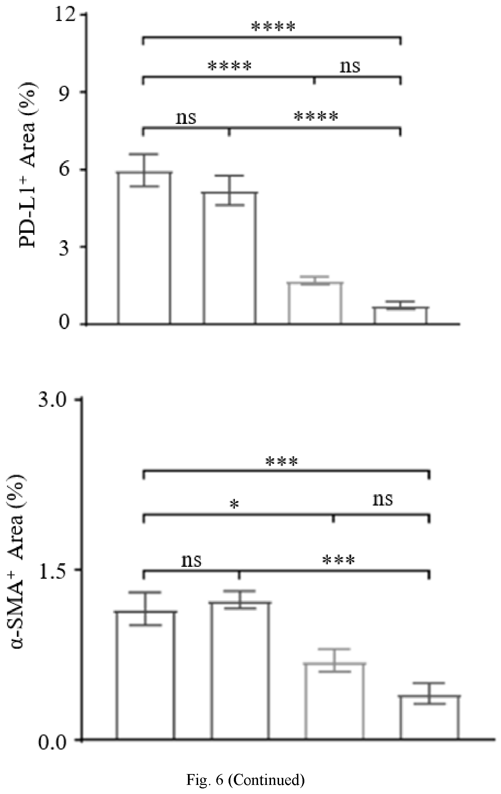

Figure 6 shows the Western blot of PD-L1 expression and IHC of SMA in each group after administration.

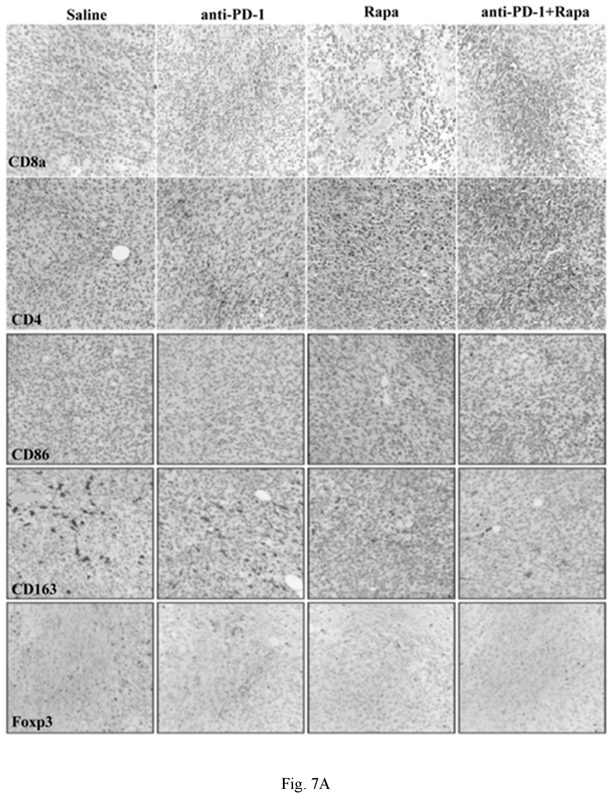

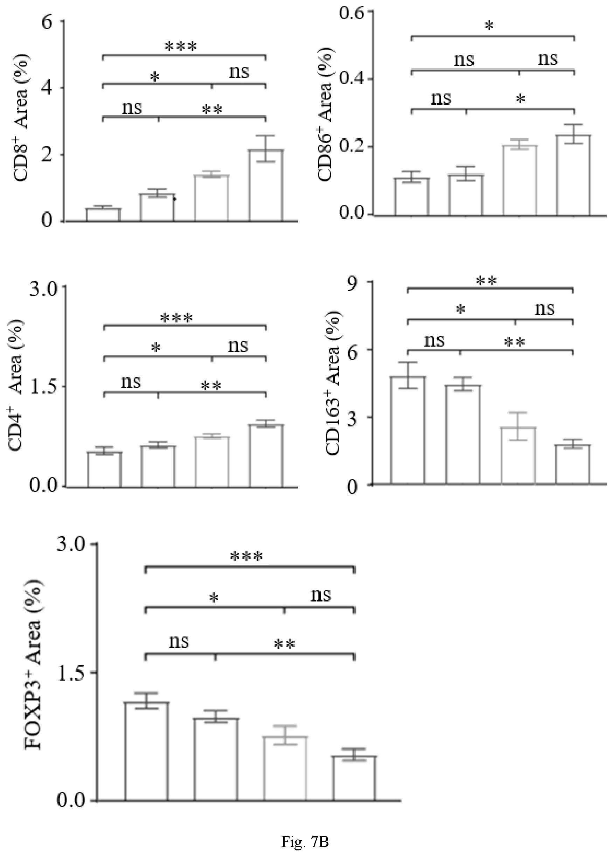

Figure 7 shows the investigation of immune cells (CD8+ T cells, CD4+ T cells, M1/M2 macrophages, regulatory T cells) by rapamycin solution combined with anti-PD-1 antibody.

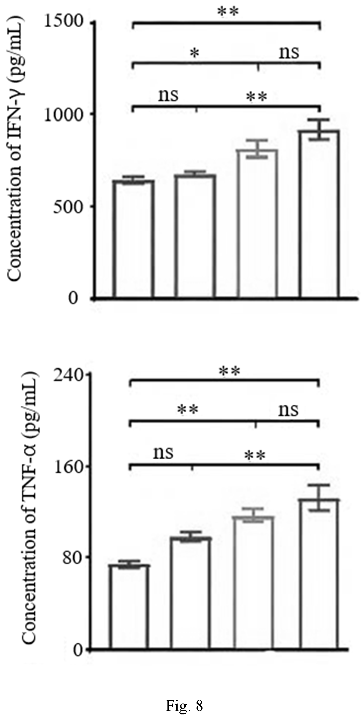

Figure 8 shows the ELISA results of rapamycin solution combined with anti-PD-1 antibody (IFN-$\gamma$, TNF-$\alpha$).

**Detailed Description of the Embodiments**

Example 1 Preparation of Rapamycin Liposomes

Rapamycin liposomes were prepared by the anhydrous ethanol injection method. The Rapamycin liposomes were characterized by measuring particle size, potential, drug loading, encapsulation efficiency, and structure via TEM. The detailed method is as follows:

100 mg of rapamycin, 900 mg of phospholipid, and 100 mg of cholesterol (stabilizer) were dissolved in 7 mL of dichloromethane (organic phase solvent) to obtain an organic mixture; the organic mixture was added dropwise into PBS (aqueous phase solvent) at a rate of 1-10 drops/min and stirred for 60 min at a temperature $\leq$ 40 °C with a stirring speed of 600 rpm to obtain a primary emulsion; the primary emulsion was homogenized six times on a homogenizer at a homogenization pressure of 900 bar, then mixed with 5% trehalose (lyoprotectant), filtered through a microporous membrane with a pore size of 0.22 $\mu$m for sterilization, and lyophilized to obtain rapamycin liposome lyophilized powder preparation.

Example 2 Preparation of Rapamycin Micelles

Rapamycin micelles were prepared by the organic solvent injection method. The Rapamycin micelles were characterized by measuring particle size, potential, drug loading, encapsulation efficiency, and structure via TEM. The detailed method is as follows:

100 mg of rapamycin raw material and 900 mg of mPEG-PLA block copolymer were dissolved in 7 mL of acetone to form an organic phase; the organic phase was drawn into a syringe and added dropwise at a speed of 5 drops/min into 100 mL of PBS buffer under stirring at 500 rpm, then stirred at 800 rpm at room temperature for 120 min; the organic solvent was recovered under reduced pressure at 40 °C; after centrifugation at 4000 rpm for 30 min, the supernatant was collected and sterilized by filtration through a microporous membrane with a pore size of 0.22 $\mu$m to obtain the micelle solution; lactose was added to the micelle solution, which was then sterile filtered through a microporous membrane with a pore size of 0.22 μm, and lyophilized to obtain the rapamycin micelle formulation.

Example 3 Preparation of Rapamycin Self-Microemulsifying Injection (LPMSY)

(1) 0.5 ml of anhydrous ethanol and 0.5 ml of polyethylene glycol 300 were mixed uniformly to obtain a mixture solution, rapamycin was added to the mixture solution, and then 0.5 μg of antioxidant and 2.5 μg of anhydrous citric acid were added to obtain a 20 mg/ml drug stock solution;
(2) a diluent was prepared: 0.8 ml of polyethylene glycol-10 hydroxystearate, 0.2 ml of anhydrous ethanol, and 1.0 ml of water for injection were measured, mixed uniformly, then added 0.004 g of a metal chelating agent, and mixed uniformly to obtain a diluent;
(3) the drug stock solution and the diluent were filtered and sterilized separately, then hermetically stored at 4 °C, and the drug stock solution was diluted to a desired concentration with the diluent prior to use to obtain a rapamycin self-microemulsifying injection.

Example 4 In Vitro Cytotoxicity Assay

Murine colorectal cancer cells (CT-26) were used. The in vitro cytotoxicity of rapamycin injection was determined by the 3-(4,5-Dimethylthiazol-2-yl)-2,5-diphenyltetrazolium bromide reduction method (MTT assay), and the IC50 was calculated.

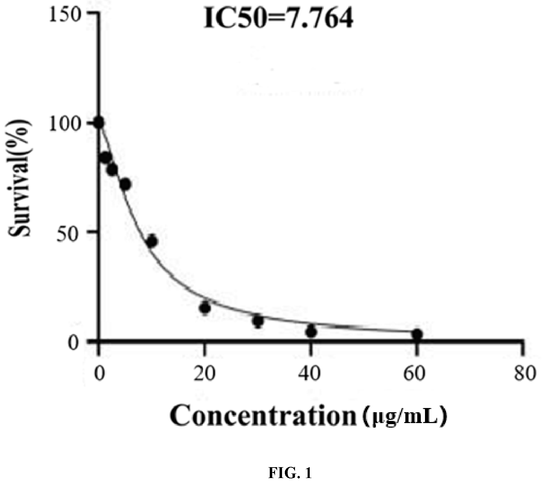

CT-26 cells were seeded in 96-well plates at $3.5 \times 10^3$ cells per well, with 6 replicate wells per concentration. After cell adhesion, the medium was replaced with serum-free medium and incubated overnight. The medium was then replaced with drug-containing medium. The concentrations of rapamycin injection were set as follows: 60, 40, 30, 20, 10, 5, 2.5, 1.25, and 0 $\mu g \cdot mL^{-1}$. After further incubation for 48 h, the medium was removed and replaced with serum-free medium containing 0.5 $mg \cdot mL^{-1}$ MTT, followed by incubation for 4 h. The medium was then removed, 100 μL of DMSO was added, and the absorbance was measured at a wavelength of 490 nm using a microplate reader after thorough mixing. Cell viability and IC50 were statistically analyzed using graphPrism. The results in Figure 1 show that the median lethal dose of rapamycin injection (LPMSY-01) against CT-26 cells is 7.764 μg/mL.

Example 5 Animal Experiments

1. Establishment of transplanted tumor mouse model

CT-26 cells (MSS murine colorectal cancer cells) in the logarithmic growth phase were harvested, counted, and resuspended in serum-free 1640 medium at a cell density of $5.0 \times 10^6$ cells/mL. The cells were placed in an ice box, and the cell suspension was drawn using a 1 mL syringe and subcutaneously injected into the right anterior axilla of BALB/c mice. Each animal was inoculated with 200 μL ($1 \times 10^6$ cells/mouse) to establish the CT-26 xenograft tumor model. The general condition of the animals was observed regularly. Tumor diameter was measured with an electronic vernier caliper, tumor volume was calculated, and tumor growth was monitored. Administration was initiated when tumor volume reached 100 mm$^3$. In the experiments, mice were divided into 4 groups (n = 6 per group). Detailed grouping and administration regimens are shown in Table 1. After the start of the experiment, tumor diameter was measured twice weekly, tumor volume was calculated, and animal body weight was weighed and recorded simultaneously.

The formula for calculating tumor volume (TV) is as follows:

$$\mathbf{TV\ (mm^3) = l \times w2\ /\ 2}$$

wherein 1 represents the tumor long diameter (mm); w represents the tumor short diameter (mm).

2. Establishment of mouse model of colorectal cancer liver metastasis

CT-26 cells (MSS murine colorectal cancer cells) in the logarithmic growth phase were harvested, counted, and resuspended in serum-free 1640 medium at a cell density of $2.0 \times 10^7$ cells/mL. The cells were placed in an ice box, and the cell suspension was drawn by using an insulin syringe, each animal was inoculated with 50 μL ($1 \times 10^6$ cells per mouse), and the colorectal cancer liver metastasis model was established by via splenic injection. The general condition of the animals was observed regularly, and tumor metastasis was monitored. Administration was started on the second day after model establishment. In the experiments, mice were divided into 4 groups (n = 6 per group). Detailed grouping and administration regimens are shown in Table 1. After the start of the experiment, tumor diameter was measured twice weekly, tumor volume was calculated, and animal body weight was weighed and recorded simultaneously.

3. Experimental Grouping and Administration Regimen

First, the therapeutic effect of rapamycin liposomes at different concentrations on CT-26 (MSS-type CRC cell) tumor-bearing mice was investigated. Compared with the saline group, rapamycin liposomes significantly inhibited tumor growth , with a statistically significant difference. The tumor inhibition rates of 1, 2, and 4 mg/kg rapamycin liposomes were 47.77%, 50.82%, and 52.98%, respectively.

The therapeutic effect of rapamycin injection solution (LPMSY-01, prepared according to Example 3) combined with PD-1 monoclonal antibody in mice with microsatellite stable colorectal cancer was preliminarily studied. Previous studies confirmed that 2 mg/kg rapamycin administered via tail vein injection exhibited favorable anti-colorectal cancer activity; thus, this dose of 2 mg/kg was used in the present study.

Table 1. Administration regimens for pharmacodynamic studies in transplanted tumor nude mouse model and liver metastasis model

| Group | Dosage | Administration Volume (mL·kg$^{-1}$) | Route of Administration | Dosing Frequency |
|---|---|---|---|---|
| blank vehicle (negative control) | | 10 | i.p. | |
| LPMSY-01 | 2 mg/kg | 10 | i.p. | once every 2 days |
| PD-1 monoclonal antibody | 200 μg | 10 | i.p. | once every 3 days |
| LPMSY-01+ PD-1 monoclonal antibody | 2 mg/kg+ 200 μg | 10 | i.p. | the same as above |

4. Preparation of administration formulations

① Preparation of blank vehicle

0.9% normal saline was used as the vehicle control group.

② Preparation of Rapamycin injection solution (LPMSY-01)

One vial of LPMSY-01 injection (approximately 20 mg/mL) was taken and diluted with the primary diluent to obtain a solution with a concentration of 4.00 mg·mL$^{-1}$, and further diluted with normal saline to a concentration of 0.2 mg·mL$^{-1}$ for administration formulation which was prepared freshly for immediate use.

③ Preparation of administration formulation for PD-1 Monoclonal Antibody (BioXcell, inVivoMab anti-mouse PD-1 (CD279))

The stock concentration of PD-1 monoclonal antibody was 9.78 mg·mL$^{-1}$. It was first diluted with normal saline to a concentration of 1.00 mg·mL$^{-1}$, and each mouse was administered 200 μL (i.e., 200 μg per mouse). The formulation was prepared freshly for immediate use.

5. Evaluation Criteria

For xenograft tumor models, the relative tumor proliferation rate T/C (%) is recommended as the evaluation index. In principle, the evaluation criteria are as follows:
T/C (%) > 40%: ineffective; T/C (%) ≤ 40% with statistical significance (P < 0.05): effective.

The results showed that combination administration exerted a significant inhibitory effect on xenograft tumors, with a T/C (%) of 31.21%, indicating effective tumor inhibition, whereas single-agent administration was ineffective. The data are summarized in Table 2.

Table 2. Summary of statistical data

| Group | Tumor inhibition rate calculated from final volume | Tumor inhibition rate calculated from tumor weight | Relative tumor proliferation rate |
|---|---|---|---|
| PD-1 monoclonal antibody | 20.53% | 21.1% | 85.31% |
| LPMSY-01 | 45.67% | 48.56% | 57.03% |
| LPMSY-01 +PD-1 monoclonal antibody | 69.93% | 76.78% | 31.21% |

6. Mechanism study

Tumor tissues were harvested from mice for paraffin sectioning and protein extraction for ELISA analysis. Immunohistochemical (IHC) staining was performed on tumor tissue sections to investigate the changes in PD-L1 protein levels in tumor tissues of CT26 tumor-bearing mice after drug administration, as well as the levels of $\alpha$-SMA$^+$ cancer-associated fibroblasts, T cells, IFN-$\gamma$, and TNF-$\alpha$ in tumor tissues after drug administration. The results of Western Blot and IHC staining of tumor tissues showed that anti-PD-1 antibody had no significant effect on PD-L1 expression on the tumor surface, whereas rapamycin solution, administered either alone or in combination, downregulated PD-L1 expression on the tumor surface. In addition, IHC staining indicated that rapamycin solution, administered either alone or in combination, downregulated the activation level of $\alpha$-SMA$^+$ cancer-associated fibroblasts in tumor tissues. Following administration with anti-PD-1 antibody, the infiltration level of CD8+ T cells in tumor tissues was slightly increased, while CD4$^+$ T cells showed no obvious changes. Rapamycin, administered either alone or in combination, increased the infiltration of CD8$^+$ T cells and CD4$^+$ T cells. Following administration with anti-PD-1 antibody, no significant changes were observed in M1/M2 macrophages and regulatory T cells in tumor tissues.

Rapamycin, administered either alone or in combination, increased the infiltration of M1 macrophages and reduced the infiltration of M2 macrophages and Tregs. ELISA results demonstrated that rapamycin solution alone or in combination enhanced the secretion of IFN-$\gamma$ and TNF-$\alpha$ in tumor tissues.

## Claims

1. Use of rapamycin combined with a PD-1 monoclonal antibody in preparation of drugs for treating colorectal cancer.

2. The use according to claim 1, wherein rapamycin and the PD-1 monoclonal antibody are formulated in a single pharmaceutical composition, or rapamycin and the PD-1 monoclonal antibody are used in combination.

3. The use according to claim 2, wherein rapamycin in the drugs for treating colorectal cancer exists in a form selected from the group consisting of liposomes, micelles, and a self-microemulsifying solution.

4. The use according to claim 3, wherein the preparation method of the self-microemulsifying solution comprises:

   (1) mixing two or more components selected from anhydrous ethanol, propylene glycol, polyethylene glycol 300, and polyethylene glycol 400, adding rapamycin, and optionally one or more antioxidants selected from 2,6-di-tert-butyl-4-methylphenol, bis(3,5-di-tert-butyl-4-hydroxyphenyl)sulfide, tetrakis[$\beta$-(3,5-di-tert-butyl-4-hydroxyphenyl)propionic acid] pentaerythritol ester, BHT, $\alpha$-tocopherol, thioglycerol, and BHA, to prepare a stock solution;
   (2) preparing a diluent by using one or more surfactants, anhydrous ethanol, water, and optionally one or more metal chelating agents, wherein the surfactants are selected from polyethylene glycol stearates such as PEG-10 hydroxystearate, PEG-12 hydroxystearate, PEG-18 stearate, PEG-10 stearate, and PEG-15 stearate, and the metal chelating agents are selected from ethylenediaminetetraacetic acid, nitrilotriacetic acid, diethylenetriaminepentaacetic acid and salts thereof, citric acid, tartaric acid, gluconic acid, hydroxyethylethylenediaminetriacetic acid, polyacrylic acid, polymethacrylic acid, hydrolyzed polymaleic anhydride, fumaric acid (trans-butenedioic acid) -allyl sulfonic acid copolymer, dihydroxyethylglycine, and sodium calcium edetate;
   (3) mixing the drug stock solution and the diluent to obtain the self-microemulsifying solution.

5. The use according to claim 4, wherein the preparation method of the self-microemulsifying solution comprises:

   (1) mixing anhydrous ethanol and polyethylene glycol 300 solution uniformly to obtain a mixture solution, adding rapamycin to the mixture solution, and then adding an antioxidant and anhydrous citric acid to obtain a drug stock solution;

(2) preparing a diluent: measuring polyethylene glycol-10 hydroxystearate, anhydrous ethanol, and water for injection, mixing uniformly, then adding a metal chelating agent, and mixing uniformly to obtain a diluent;

(3) diluting the drug stock solution to a desired concentration with the diluent to obtain a rapamycin self-microemulsifying injection.

6. The use according to claim 5, wherein the preparation method of the self-microemulsifying solution comprises:

(1) mixing 0.5 ml of anhydrous ethanol and 0.5 ml of polyethylene glycol 300 uniformly to obtain a mixture solution, adding rapamycin to the mixture solution, and then adding 0.5 $\mu$g of antioxidant and 2.5 $\mu$g of anhydrous citric acid to obtain a 20 mg/ml drug stock solution;

(2) preparing a diluent: measuring 0.8 ml of polyethylene glycol-10 hydroxystearate, 0.2 ml of anhydrous ethanol, and 1.0 ml of water for injection, mixing uniformly, then adding 0.004 g of a metal chelating agent, and mixing uniformly to obtain a diluent;

(3) filtering and sterilizing the drug stock solution and the diluent separately, followed by hermetically storing them at 4 °C, and diluting the drug stock solution to a desired concentration with the diluent prior to use to obtain a rapamycin self-microemulsifying injection.

7. The use according to any one of claims 1-6, wherein the PD-1 monoclonal antibody is selected from pembrolizumab, nivolumab, sintilimab, toripalimab, tislelizumab, camrelizumab and anti-mouse PD-1 CD279 monoclonal antibody from BioXcell, inVivoMab.

8. The use according to any one of claims 1-7, wherein the colorectal cancer is microsatellite stable colorectal cancer.

9. The use according to any one of claims 1-8, wherein the weight ratio of rapamycin to the PD-1 monoclonal antibody in the drugs is 1:1 to 4:1.

10. The use according to claim 9, wherein the weight ratio of rapamycin to the PD-1 monoclonal antibody in the drugs is 1:1 to 2:1.

11. The use according to any one of claims 1-9, wherein the weight ratio of rapamycin to the PD-1 monoclonal antibody in the drugs is 1:1 to 1:20.

12. A drug for treating colorectal cancer, **characterized in that** the drug comprises rapamycin and a PD-1 monoclonal antibody.

13. The drug according to claim 12, wherein rapamycin and the PD-1 monoclonal antibody are formulated in a single pharmaceutical composition, or rapamycin and the PD-1 monoclonal antibody are provided separately.

14. The drug according to claim 13, wherein rapamycin exists in a form selected from the group consisting of liposomes, micelles, and a self-microemulsifying solution.

15. The drug according to claim 14, wherein the preparation method of the self-microemulsifying solution comprises:

(1) mixing two or more components selected from anhydrous ethanol, propylene glycol, polyethylene glycol 300, and polyethylene glycol 400, adding rapamycin, and optionally one or more antioxidants selected from 2,6-di-tert-butyl-4-methylphenol, bis(3,5-di-tert-butyl-4-hydroxyphenyl)sulfide, tetrakis[$\beta$-(3,5-di-tert-butyl-4-hydroxyphenyl)propionic acid] pentaerythritol ester, BHT, $\alpha$-tocopherol, thioglycerol, and BHA, to prepare a stock solution;

(2) preparing a diluent by using one or more surfactants, anhydrous ethanol, water, and optionally one or more metal chelating agents, wherein the surfactants are selected from polyethylene glycol stearates such as PEG-10 hydroxystearate, PEG-12 hydroxystearate, PEG-18 stearate, PEG-10 stearate, and PEG-15 stearate, and the metal chelating agents are selected from ethylenediaminetetraacetic acid, nitrilotriacetic acid, diethylenetriaminepentaacetic acid and salts thereof, citric acid, tartaric acid, gluconic acid, hydroxyethylethylenediaminetriacetic acid, polyacrylic acid, polymethacrylic acid, hydrolyzed polymaleic anhydride, fumaric acid (trans-butenedioic acid) -allyl sulfonic acid copolymer, dihydroxyethylglycine, and sodium calcium edetate;

(3) mixing the drug stock solution and the diluent to obtain the self-microemulsifying solution.

16. The drug according to claim 15, wherein the preparation method of the self-microemulsifying solution comprises:

(1) mixing anhydrous ethanol and polyethylene glycol 300 solution uniformly to obtain a mixture solution, adding rapamycin to the mixture solution, and then adding an antioxidant and anhydrous citric acid to obtain a drug stock solution;
(2) preparing a diluent: measuring polyethylene glycol-10 hydroxystearate, anhydrous ethanol, and water for injection, mixing uniformly, then adding a metal chelating agent, and mixing uniformly to obtain a diluent;
(3) diluting the drug stock solution to a desired concentration with the diluent to obtain a rapamycin self-microemulsifying injection.

17. The drug according to claim 16, wherein the preparation method of the self-microemulsifying solution comprises:

(1) mixing 0.5 ml of anhydrous ethanol and 0.5 ml of polyethylene glycol 300 uniformly to obtain a mixture solution, adding rapamycin to the mixture solution, and then adding 0.5 $\mu$g of antioxidant and 2.5 $\mu$g of anhydrous citric acid to obtain a 20 mg/ml drug stock solution;
(2) preparing a diluent: measuring 0.8 ml of polyethylene glycol-10 hydroxystearate, 0.2 ml of anhydrous ethanol, and 1.0 ml of water for injection, mixing uniformly, then adding 0.004 g of a metal chelating agent, and mixing uniformly to obtain a diluent;
(3) filtering and sterilizing the drug stock solution and the diluent separately, followed by hermetically storing them at 4 °C, and diluting the drug stock solution to a desired concentration with the diluent prior to use to obtain a rapamycin self-microemulsifying injection.

18. The drug according to any one of claims 12-17, wherein the PD-1 monoclonal antibody is selected from pembrolizumab, nivolumab, sintilimab, toripalimab, tislelizumab, camrelizumab and anti-mouse PD-1 CD279 monoclonal antibody from BioXcell, inVivoMab.

19. The drug according to any one of claims 12-18, wherein the colorectal cancer is microsatellite stable colorectal cancer.

20. The drug according to any one of claims 12-19, wherein the weight ratio of rapamycin to the PD-1 monoclonal antibody in the drug is 1:1 to 4:1.

21. The drug according to claim 20, wherein the weight ratio of rapamycin to the PD-1 monoclonal antibody in the drug is 1:1 to 2:1.

22. The drug according to any one of claims 12-19, wherein the weight ratio of rapamycin to the PD-1 monoclonal antibody in the drug is 1:1 to 1:20.

23. A method of treating colorectal cancer, comprising administering to a subject in need thereof rapamycin in combination with a PD-1 monoclonal antibody.

24. The method according to claim 23, wherein rapamycin and the PD-1 monoclonal antibody are formulated in a single pharmaceutical composition, or rapamycin and the PD-1 monoclonal antibody are used in combination.

25. The method according to claim 23 or 24, wherein the PD-1 monoclonal antibody is selected from pembrolizumab, nivolumab, sintilimab, toripalimab, tislelizumab, camrelizumab and anti-mouse PD-1 CD279 monoclonal antibody from BioXcell, inVivoMab.

26. The method according to claim 23 or 24, wherein rapamycin in the drug for treating colorectal cancer exists in a form selected from the group consisting of liposomes, micelles, and a self-microemulsifying solution.

27. The method according to claim 26, wherein the preparation method of the self-microemulsifying solution comprises:

(1) mixing two or more components selected from anhydrous ethanol, propylene glycol, polyethylene glycol 300, and polyethylene glycol 400, adding rapamycin, and optionally one or more antioxidants selected from 2,6-di-tert-butyl-4-methylphenol, bis(3,5-di-tert-butyl-4-hydroxyphenyl)sulfide, tetrakis[$\beta$-(3,5-di-tert-butyl-4-hydroxyphenyl)propionic acid] pentaerythritol ester, BHT, $\alpha$-tocopherol, thioglycerol, and BHA, to prepare a stock solution;
(2) preparing a diluent by using one or more surfactants, anhydrous ethanol, water, and optionally one or more metal chelating agents, wherein the surfactants are selected from polyethylene glycol stearates such as PEG-10 hydroxystearate, PEG-12 hydroxystearate, PEG-18 stearate, PEG-10 stearate, and PEG-15 stearate, and the metal chelating agents are selected from ethylenediaminetetraacetic acid, nitrilotriacetic acid, diethylenetriaminepentaacetic acid and salts thereof, citric acid, tartaric acid, gluconic acid, hydroxyethylethylenediaminetriacetic acid, polyacrylic acid, polymethacrylic acid, hydrolyzed polymaleic anhydride, fumaric acid (trans-butenedioic acid) -allyl sulfonic acid copolymer, dihydroxyethylglycine, and sodium calcium edetate;

(3) mixing the drug stock solution and the diluent to obtain the self-microemulsifying solution.

28. The method according to claim 27, wherein the preparation method of the self-microemulsifying solution comprises:

(1) mixing anhydrous ethanol and polyethylene glycol 300 solution uniformly to obtain a mixture solution, adding rapamycin to the mixture solution, and then adding an antioxidant and anhydrous citric acid to obtain a drug stock solution;

(2) preparing a diluent: measuring polyethylene glycol-10 hydroxystearate, anhydrous ethanol, and water for injection, mixing uniformly, then adding a metal chelating agent, and mixing uniformly to obtain a diluent;

(3) diluting the drug stock solution to a desired concentration with the diluent to obtain a rapamycin self-microemulsifying injection.

29. The method according to claim 28, wherein the preparation method of the self-microemulsifying solution comprises:

(1) mixing 0.5 ml of anhydrous ethanol and 0.5 ml of polyethylene glycol 300 uniformly to obtain a mixture solution, adding rapamycin to the mixture solution, and then adding 0.5 $\mu$g of antioxidant and 2.5 $\mu$g of anhydrous citric acid to obtain a 20 mg/ml drug stock solution;

(2) preparing a diluent: measuring 0.8 ml of polyethylene glycol-10 hydroxystearate, 0.2 ml of anhydrous ethanol, and 1.0 ml of water for injection, mixing uniformly, then adding 0.004 g of a metal chelating agent, and mixing uniformly to obtain a diluent;

(3) filtering and sterilizing the drug stock solution and the diluent separately, followed by hermetically storing them at 4 °C, and diluting the drug stock solution to a desired concentration with the diluent prior to use to obtain a rapamycin self-microemulsifying injection.

30. The method according to any one of claims 23-29, wherein the colorectal cancer is microsatellite stable colorectal cancer.

31. The method according to any one of claims 23-30, wherein the weight ratio of rapamycin to the PD-1 monoclonal antibody in the drug is 1:1 to 4:1.

32. The method according to claim 31, wherein the weight ratio of rapamycin to the PD-1 monoclonal antibody in the drug is 1:1 to 2:1.

33. The method according to any one of claims 23-30, wherein the weight ratio of rapamycin to the PD-1 monoclonal antibody in the drug is 1:1 to 1:20.

34. The method according to any one of claims 23-30, wherein the dosage of rapamycin in the drug is 0.5-4 mg/kg, and the dosage of the PD-1 monoclonal antibody is 0.5-80 mg/kg.

FIG. 1

The first animal experiment

The second animal experiment

**FIG.2**

**FIG.3**

**FIG.4**

FIG.5

FIG.6

Fig. 6 (Continued)

Fig. 6 (Continued)

Fig. 7A

Fig. 7B

Fig. 8

## INTERNATIONAL SEARCH REPORT

| | International application No. |
|---|---|
| | **PCT/CN2023/143213** |

| A. CLASSIFICATION OF SUBJECT MATTER |
|---|
| A61K 39/395(2006.01)i; A61K 31/436(2006.01)i; A61K 9/107(2006.01)i; A61K 9/127(2006.01)i; A61P 35/00(2006.01)i |

According to International Patent Classification (IPC) or to both national classification and IPC

| B. FIELDS SEARCHED |
|---|

| Minimum documentation searched (classification system followed by classification symbols) |
|---|
| IPC:A61K,A61P |

Documentation searched other than minimum documentation to the extent that such documents are included in the fields searched

| Electronic data base consulted during the international search (name of data base and, where practicable, search terms used) |
|---|
| CNTXT, WPABSC, WPABS, ENTXTC, ENTXT, 超星读秀学术, CHAOXING DUXIU SCHOLAR, CNKI, 万方, WANFANG, ISI web of knowledge, Elsevier Science Direct, Springerlink, pubmed, 百度, Baidu, 百度学术, Baidu Scholar, patentics: 雷帕霉素, PD-1, PD1, 单抗, 单克隆抗体, 抗体, 抑制剂, 联合, 结直肠癌, rapamycin, RAPA, colorectal cancer, CRC, mTOR, inhibitor, antibody, combine, 自微乳, self-microemulsifying |

| C. DOCUMENTS CONSIDERED TO BE RELEVANT |
|---|

| Category* | Citation of document, with indication, where appropriate, of the relevant passages | Relevant to claim No. |
|---|---|---|
| PX | CN 116898964 A (GUANGZHOU PENGXING PHARMACEUTICAL TECHNOLOGY CO., LTD.) 20 October 2023 (2023-10-20) claims 1-22 | 1-34 |
| X | YANG, M. et al. "TOX Acts as a Tumor Suppressor by Inhibiting mTOR Signaling in Colorectal Cancer" *Front. Immunol.*, Vol. vol. 12, 09 April 2021 (2021-04-09), Article 647540, pp. 1-14 | 1-2, 7-13, 18-25, 30-34 |
| Y | YANG, M. et al. "TOX Acts as a Tumor Suppressor by Inhibiting mTOR Signaling in Colorectal Cancer" *Front. Immunol.*, Vol. vol. 12, 09 April 2021 (2021-04-09), Article 647540, pp. 1-14 | 3-11, 14-22, 26-34 |
| Y | CN 114366715 A (YAN, Pengke) 19 April 2022 (2022-04-19) claims 1-10, and description, embodiments 1-6 | 3-11, 14-22, 26-34 |

| ✓ Further documents are listed in the continuation of Box C. | ✓ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **08 May 2024** | **15 May 2024** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2023/143213** |

**C. DOCUMENTS CONSIDERED TO BE RELEVANT**

| Category* | Citation of document, with indication, where appropriate, of the relevant passages | Relevant to claim No. |
| --- | --- | --- |
| Y | EP 4218721 A1 (YAN, Pengke) 02 August 2023 (2023-08-02) claims 1-10, and description, embodiments 1-6 | 3-11, 14-22, 26-34 |
| A | WO 2011151704 A2 (FRESENIUS KABI ONCOLOGY LTD.) 08 December 2011 (2011-12-08) entire document | 1-34 |
| A | BAI, X. et al. "Improvement of PD-1 Blockade Efficacy and Elimination of Immune-Related Gastrointestinal Adverse Effect by mTOR Inhibitor" *Front. Immunol.*, Vol. vol. 12, 20 December 2021 (2021-12-20), Article 793831, pp. 1-14 | 1-34 |
| A | FENG, Y. et al. "Programmed Death-Ligand 1 and Mammalian Target of Rapamycin Signaling Pathway in Locally Advanced Rectal Cancer" *Discover Oncology*, Vol. vol. 13, 14 February 2022 (2022-02-14), Article 10 | 1-34 |

Form PCT/ISA/210 (second sheet) (July 2022)

## INTERNATIONAL SEARCH REPORT

<table>
<tr><td>International application No.</td></tr>
<tr><td>**PCT/CN2023/143213**</td></tr>
</table>

**Box No. II   Observations where certain claims were found unsearchable (Continuation of item 2 of first sheet)**

This international search report has not been established in respect of certain claims under Article 17(2)(a) for the following reasons:

1. ☑ Claims Nos.: **23-34**
   because they relate to subject matter not required to be searched by this Authority, namely:

   Claims 23-34 relate to a method for treating colorectal cancer, which falls within disease treatment methods as defined in PCT Rule 39.1(iv). Therefore, a search is carried out on the basis of the corresponding pharmaceutical use of a pharmaceutical composition of rapamycin and a PD-1 monoclonal antibody.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.

**PCT/CN2023/143213**

| Patent document cited in search report | | | Publication date (day/month/year) | Patent family member(s) | | | Publication date (day/month/year) |
|---|---|---|---|---|---|---|---|
| CN | 116898964 | A | 20 October 2023 | None | | | |
| CN | 114366715 | A | 19 April 2022 | None | | | |
| EP | 4218721 | A1 | 02 August 2023 | AU | 2023200353 | A1 | 17 August 2023 |
| | | | | US | 2023241040 | A1 | 03 August 2023 |
| WO | 2011151704 | A2 | 08 December 2011 | AU | 2011260016 | A1 | 10 January 2013 |
| | | | | AU | 2011260016 | B2 | 22 August 2013 |
| | | | | US | 2011301189 | A1 | 08 December 2011 |
| | | | | WO | 2011151704 | A3 | 01 March 2012 |
| | | | | JP | 2013527223 | A | 27 June 2013 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202311128066
- CN 201910918643
- CN 202011065631
- CN 202210106827